# EUROPEAN PATENT SPECIFICATION

(11) **EP 2 014 771 B1**
(45) Date of publication and mention of the grant of the patent: **03.12.2014**
(21) Application number: 07110957.3
(22) Date of filing: 25.06.2007
(51) Int. Cl.: C12P 19/40

(54) **Continuous enzymatic hydrolysis process**
Kontinuierliches enzymatisches Hydrolyseverfahren
Procédé d'hydrolyse enzymatique continu

(43) Date of publication of application: 14.01.2009
(73) Proprietor: Anadys Pharmaceuticals, Inc., 1 DNA Way South San Francisco, CA 94080 (US)
(72) Inventor: Gallou, Fabrice., CH- 4051 Basel (CH); Beney, Pascal., CH- 4312 Magden (CH)
(74) Representative: Schiweck, Weinzierl & Koch

(56) References cited:
- WO-A-2005/121162
- FR-A- 2 648 147
- TERRENI, MARCO ET AL: "Regioselective enzymatic hydrolysis of acetylated pyranoses and pyranosides using immobilized lipases . An easy chemoenzymatic synthesis of .alpha.- and .beta.-D-glucopyranose acetates bearing a free secondary C-4 hydroxyl group" CARBOHYDRATE RESEARCH , 337(18), 1615-1621 CODEN: CRBRAT; ISSN: 0008-6215, 2002, XP004390860
- PANERO, JULIETA ET AL: "Microbial Hydrolysis of Acetylated Nucleosides" BIOTECHNOLOGY LETTERS , 28(14), 1077-1081 CODEN: BILED3; ISSN: 0141-5492, 2006, XP019391541

## Description

The present invention relates to an improved process for the regioselective enzymatic hydrolysis of alcohol groups protected e.g. as esters or amino-acid esters or phosphate groups.

WO05/121162 describes certain D-ribofuranosyl compounds which are prepared by selective hydrolysis at the 5'-group of the ribose moiety of the protected alcohol group. However, the enzymatic hydrolysis process as described in WO05/121162 has shortcomings inherent to the heterogeneous process such as, e.g. a long reaction time, limited selectivity in the hydrolysis, requirement of a large vessel for each batch, several filtration step and no easy recycling of the enzyme.

The present invention now provides an improved process for the regioselective enzymatic hydrolysis which overcomes many of the shortcomings of previously used regioselective enzymatic hydrolysis processes. In accordance with the present invention, it has surprisingly been found that by using a continuous process for the regioselective enzymatic hydrolysis of substrates having more than one hydrolysable groups, higher selectivity of the hydrolysis and lower impurities with undesired hydrolysis product can be achieved. Furthermore, such a continuous process constitutes a long-term and economic solution to the previously used long and costly batch process having a low throughput. The process of the invention can dramatically reduce the cycle time and minimize the impact of the overall low volume performance as compared to e.g. the process described in WO05/121162. This is particularly relevant for scale-ups of the process where the volumes of the process increase.

Accordingly, in its broadest aspect, the present invention provides a process for the regioselective enzymatic hydrolysis of a substrate comprising more than one hydrolysable groups wherein said enzymatic hydrolysis is performed in a continuous mode.

In the continuous process in accordance with the present invention, typically a buffered solution of adduct is passed through an immobilized enzyme. The term "continuous" in accordance with the present invention refers to a process continuously (without interruptions) passed through the column. After suitable residence time, the adduct is fully hydrolyzed as can be monitored in situ, e.g., via pH monitoring of the solution comprising the product collected after the column and subsequently extracted. Critical parameters of the of the continuous process need to be adjusted individually depending for instance on the substrate, enzyme etc. and can be determined empirically case by case. Such parameters include for instance the residence time, the packing of the column, the optimum pH , the temperature, the concentration of adduct, the choice of organic solvent. The residence time, for instance, is adjusted such that the adduct is optimally hydrolyzed, i.e. with high selectivity and rapid conversion and may typically be from 0.1 min to 300 min. The residence time, for instance, depends on the enzyme activity, the temperature, pH, solvent system and is adjusted such that it allows for continuous processing, and optimal hydrolysis as defined above. The pH and the temperature are usually chosen in accordance with the condition the enzyme needs for the hydrolysis reaction. For instance, the pH may be in the range between e.g. 5 and 8 or, e.g. 5.5 to 7.5. The temperature may be in the range of e.g. 15°C to 70°C.

Any buffer suitable for the enzymatic reaction may be used, such as e.g. a phosphate buffer, an ammonium buffer, a carbonate buffer, an acetate buffer.

Furthermore, a suitable organic component can be used to allow for complete solubilization of the adduct and product. Typical organic components include e.g. acetone, methylethylketone, methylisobutylketone, methanol, methanol, ethanol, isopropanol, n-butanol, 3-methyl-1-butanol, 2-methoxyethanol, 2-ethoxyethanol, ter-butyl methylether, tetrahydrofuran, 2-methyltetrahydrofuran, dioxane, acetonitrile, dichloromethane, dimethylformamide, dimethylsulfoxide, ionic liquids, compressed gases such as carbon dioxide, water, or the like, and mixtures thereof. Other alcohols, ethers, ketones can also be imagined.

Additionally, an additives can be used, e.g., to enhance the rate of the reaction. Typical additives include for instance PEG (1% to 10%), NaCl, Na2SO4, FeCl3. Suitable concentration of such additives can be determined empirically and may typically be in a concentration range of 0.05M to 1 M. Conveniently, the additives can be added to the solution of adduct.

The enzyme is immobilized on a physical support, e.g. a solid support. A physical support for the immobilized enzyme suitable for the present invention includes e.g. a column, a continuous stirred tank, a packed-bed reactor, a membrane reactor, a membrane. Any enzyme suitable for hydrolysis can be used in accordance with the present invention, such as e.g. Esterases, Hydrolases, Lipases.

Suitable substrates for the processes of the present invention contain at least two groups which is hydrolysable, i.e. e.g. two acetates, benzoates. Typical examples of such groups are alcohol groups protected as esters, amino acid esters, phosphates. In one embodiment the substrates are pyranosides or furanosides.

In accordance with one aspect of the present invention, the substrate is a compound as generally (without stereochemistry) depicted by Formula (1) to (21) 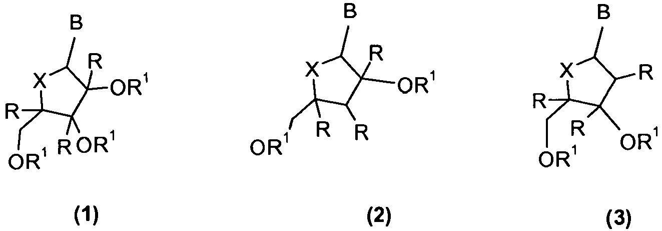 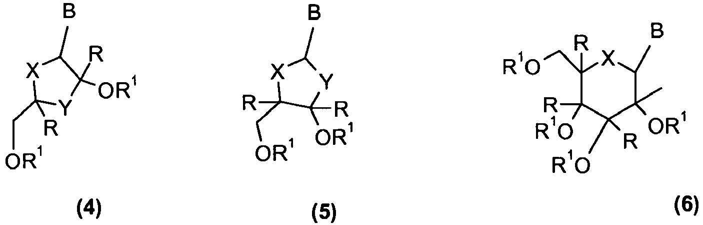 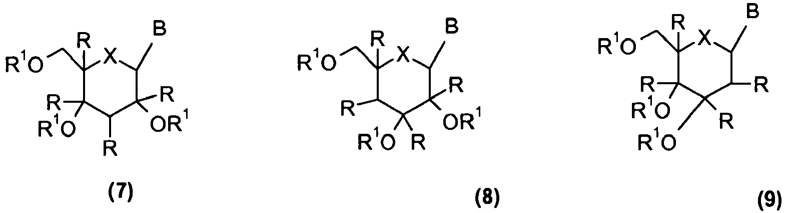 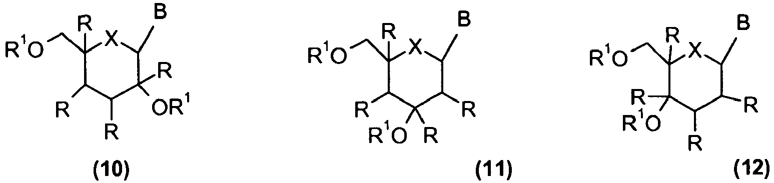 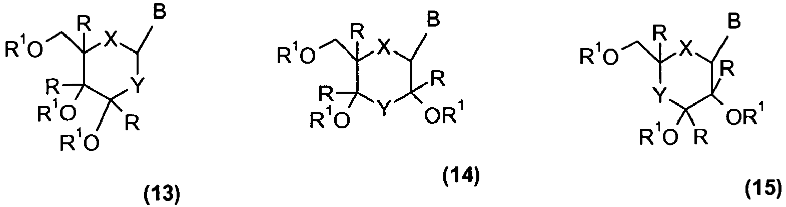 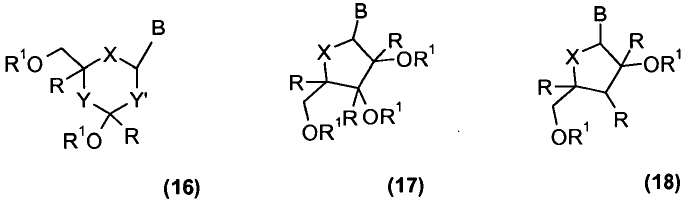 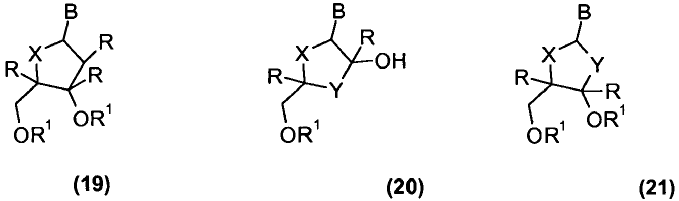 wherein R is independently H, alkyl, hydroxy, hydroxyalkyl, -NR'R", -SR"', halogeno; R' and R" are independently alkyl, -SR"', -SOR"', -SO₂R"'; R"' is independently H, alkyl, aryl; R¹ is independently H, -C(O)R³, a racemic, L-, or D- amino acid group -C(O)CH₂NHR⁴, - C(O)CH(C₁₋₆ alkyl)NHR⁴, phosphate; R³ is a C₁₋₁₈ alkyl; R⁴ is H, -C(O)CH(C₁₋₆ alkyl)NH₂, or - C(O)CH(CH₂-aryl)NH₂; B is a nucleobase; X, Y and Y' are independently -CH2-, -CHR'-, - CR'R"- or O, NR"', S wherein R' and R" are independently alkyl and R"' is H or alkyl or CO(Z), Z being O-alkyl or NH-Alkyl or N-Alkyl2; and R² is H, -C(O)CH(C₁₋₆ alkyl)NH₂, or - C(O)CH(CH₂-aryl)NH₂.

The term "alkyl", as used herein, includes saturated monovalent hydrocarbon radicals having straight, branched, or cyclic moieties (including fused and bridged bicyclic and spirocyclic moieties), or a combination of the foregoing moieties. Examples of preferred alkyl groups include C₁₋₁₈ or C₁₋₁₂ alkyls. An aryl group may be unsubstituted or substituted at any position. Typically, it carries 0, 1, 2 or 3 substituents. In another preferred embodiment the alkyl is lower alkyl, such as e.g. C₁₋₆ more preferably C₁₋₄. Particularly preferred are methyl, ethyl, propyl, isopropyl, butyl, sec.- or tert.-butyl, n- or branched pentyl. An alkyl group may be unsubstituted or substituted at any position. Typically, it carries 0, 1, 2 or 3 substituents.

The term "alkenyl", as used herein, includes alkyl moieties having at least one carbon-carbon double bond wherein alkyl is as defined above and including E and Z isomers of said alkenyl moiety. The term "alkynyl", as used herein, includes alkyl moieties having at least one carbon-carbon triple bond wherein alkyl is as defined above.

The term "aryl" The term "aryl", as used herein, includes an organic radical derived from an aromatic hydrocarbon by removal of one hydrogen, and is typically a C₆₋₁₀ aryl group. An aryl group may be unsubstituted or substituted at any position. Typically, it carries 0, 1, 2 or 3 substituents. Typical examples include phenyl or naphthyl.

The term "phosphate", as used herein, includes one or several phosphate groups, e.g. -(HO(PO)OH)ₘ -(HO(PO(OH))OH), m is 0, 1 or 2 and n is 0, 1, 2, 3, 4, 5.

The alkyl or aryl groups in accordance with the present invention can also be further substituted, e.g. with one or more halo (F, Cl, Br, I) substituent or one or more of the following substituents: cyano, nitro, trifluoromethyl, trifluoromethoxy, C₁-C₆ alkyl, C₂-C₆ alkenyl, C₂-C₆ alkynyl, hydroxy, C₁-C₆ alkoxy, -NH₂, -NH-alkyl, -N(alkyl)₂, -NH-aryl, - N(alkyl)(aryl), -N(aryl)₂, -NHCHO, -NHC(O)alkyl, -NHC(O)aryl, -N(alkyl)C(O)H, - N(alkyl)C(O)alkyl, -N(aryl)C(O)H, -N(aryl)C(O)alkyl, -NHCO₂alkyl, -N(alkyl)CO₂alkyl, - NHC(O)NH₂, -N(alkyl)C(O)NH₂, -NHC(O)NH-alkyl, -NHC(O) N(alkyl)₂, -N(alkyl)C(O)NH-alkyl, N(alkyl)C(O) N(alkyl)₂, -NHSO₂-alkyl, -N(alkyl)SO₂-alkyl, -C(O)alkyl, -C(O)aryl, -OC(O)alkyl, - OC(O)aryl, -CO₂-alkyl, -CO₂-aryl, -CO₂H, -C(O)NH₂, -C(O)NH-alkyl, -C(O)N(alkyl)₂, -C(O)NH-aryl, -C(O)N(aryl)₂, -C(O)N(alkyl)(aryl), -S(O)alkyl, -S(O)aryl, -SO₂alkyl, -SO₂aryl, -SO₂NH₂,-SO₂NH-alkyl, and -SO₂N(alkyl)₂.

The term "halo" or "halogeno", as used herein, refers to F, CI, Br or I.

The term nucleobase in the context of the present invention refers to any base suitable to be incorporated into a nucleic acid, as e.g. exemplified in WO05/121162.

In one embodiment, a hydrolysable protected primary alcohol group, e.g. an ester of a primary alcohol, is selectively hydrolyzed in the presence of on or more hydrolysable protected secondary alcohol group, e.g. an ester of a secondary alcohol.

In one preferred aspect of the present invention, the ribofuranoside is a ribofuranosylthiazolo[4,5-*d*]pyrimidine. Suitable compounds are for instance described in WO05/121162 which relates the to 3-β-D-ribofuranosylthiazolo[4,5-*d*]pyrimidine nucleosides. In one embodiment, the compound is compound 89 of WO05/121162.

Accordingly, in one preferred embodiment the substrate is a compound of Formula 22 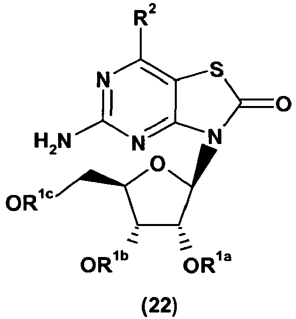 wherein:
R^{1a}, R^{1b}, and R^{1c} are independently H, -C(O)R³, a racemic, L-, or D- amino acid group - C(O)CH₂NHR⁴, -C(O)CH(C₁₋₆ alkyl)NHR⁴, or R^{1b} and R^{1c} are collectively -C(O)-, which together with the oxygen atoms forms a five-membered carbonate ring;
R² is H, OR⁵, or N(R⁶)₂; R³ is a C₁₋₁₈ alkyl; R⁴ is H, -C(O)CH(C₁₋₆ alkyl)NH₂, or -C(O)CH(CH₂-aryl)NH₂; R⁵ is independently H, C₁₋₆ alkyl, C₃₋₇ alkenyl, C₃₋₇ alkynyl, -(CR⁷R⁸)ₜ(C₆-C₁₀ aryl), - (CR⁷R⁸)ₜ(C₃-C₁₀ cycloalkyl), -(CR⁷R⁸)ₜ(C₄-C₁₀ heterocyclic), -(CR⁷R⁸)_{t>1}OH, -(CR⁷R⁸)_{t>0}CO₂C₁₋₁₈ alkyl, and -(CR⁷R⁸)_{t>0}N(R⁹)CO₂C₁₋₁₈ alkyl, and SO₂(aryl), wherein t is an integer from 0 to 6, and wherein the alkyl, alkenyl, alkynyl, aryl, cycloalkyl, and heterocyclic moieties of the foregoing groups are optionally substituted with substituents independently selected from halo, cyano, nitro, trifluoromethyl, trifluoromethoxy, C₁-C₆ alkyl, C₂-C₆ alkenyl, C₂-C₆ alkynyl, hydroxy, C₁-C₆ alkoxy, -NH₂, -NH-alkyl, -N(alkyl)₂, -NH-aryl, -N(alkyl)(aryl), -N(aryl)₂, - NHCHO, -NHC(O)alkyl, -NHC(O)aryl, -N(alkyl)C(O)H, -N(alkyl)C(O)alkyl, -N(aryl)C(O)H, - N(aryl)C(O)alkyl, -NHCO₂alkyl, -N(alkyl)CO₂alkyl, -NHC(O)NH₂, -N(alkyl)C(O)NH₂, - NHC(O)NH-alkyl, -NHC(O) N(alkyl)₂, -N(alkyl)C(O)NH-alkyl, N(alkyl)C(O) N(alkyl)₂, -NHSO₂-alkyl, -N(alkyl)SO₂-alkyl, -C(O)alkyl, -C(O)aryl, -OC(O)alkyl, -OC(O)aryl, -CO₂-alkyl, -CO₂-aryl, -CO₂H, -C(O)NH₂, -C(O)NH-alkyl, -C(O)N(alkyl)₂, -C(O)NH-aryl, -C(O)N(aryl)₂, - C(O)N(alkyl)(aryl), -S(O)alkyl, -S(O)aryl, -SO₂alkyl, -SO₂aryl, -SO₂NH₂, -SO₂NH-alkyl, and - SO₂N(alkyl)₂; R⁶ is independently H, C₁₋₆ alkyl, C₃-C₁₀ cycloalkyl, or together with nitrogen forms a 5- or 6-membered heterocyclic ring; R⁷ and R⁸ are independently H, C₁₋₆ alkyl, C₂₋₆ alkenyl, or C₂₋₆ alkynyl; and R⁹ is H, C₁₋₆ alkyl, or -CH₂-aryl; and wherein said compound comprises at least two hydrolysable groups.

In one embodiment, the invention relates to a compound of the Formula 22, wherein R² is H or OR⁵ and wherein said compound comprises at least two hydrolysable groups.

In another embodiment, the invention relates to compounds of the Formula 22 wherein R^{1a}, R^{1b}, and R^{1c} are independently H, -C(O)R³, a racemic, L-, or D-amino acid group -C(O)CH(C₁₋₆ alkyl)NH₂; R² is OR⁵; R³ is a C₁₋₁₈ alkyl; R⁵ is independently C₁₋₆ alkyl, C₃₋₇ alkenyl, C₃₋₇ alkynyl, -(CR⁷R⁸)ₜ(C₆-C₁₀ aryl), -(CR⁷R⁸)ₜ(C₄-C₁₀ heterocyclic), and -(CR⁷R⁸)_{t>0}N(R⁹)CO₂C₁₋₁₈ alkyl, wherein t is an integer from 0 to 4 unless otherwise indicated, and wherein the alkyl, alkenyl, aryl, and heterocyclic moieties of the foregoing groups are optionally substituted with 1 to 3 substituents independently selected from halo, cyano, nitro, trifluoromethyl, trifluoromethoxy, C₁-C₆ alkyl, C₂-C₆ alkenyl, C₂-C₆ alkynyl, hydroxy, C₁-C₆ alkoxy, -CO₂-alkyl, -CO₂-aryl, -OC(O)alkyl, and -OC(O)aryl; R⁷ and R⁸ are independently H, C₁₋₆ alkyl, or C₂₋₆ alkenyl; and R⁹ is H, -CH₃, or -CH₂CH₃.

In another embodiment, the invention relates to compounds of the Formula 22 wherein R^{1a}, R^{1b}, and R^{1c} are independently H, -C(O)R³, R² is H and wherein R³ is lower alkyl. In another embodiment, R^{1a}, R^{1b}, and R^{1c} are H, -C(O)R³, R² is H and wherein R³ is lower alkyl.

Examples of other substrates suitable for regioselective hydrolysis by the a continuous process in accordance with the present invention include: 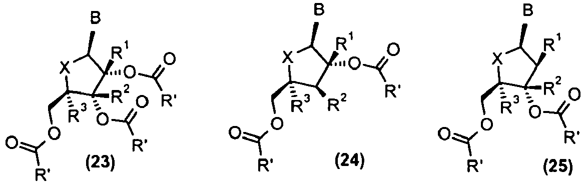 and other diastereoisomers 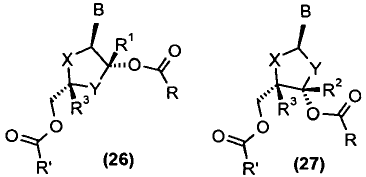 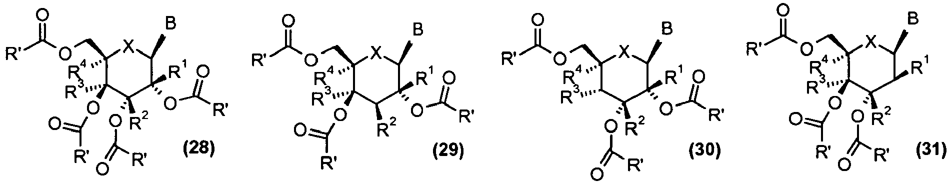 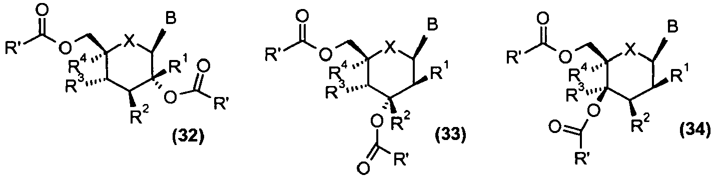 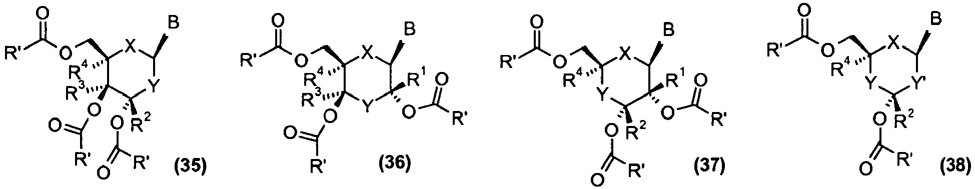 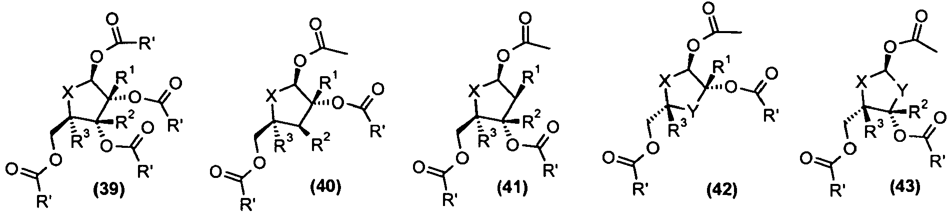 and other diastereoisomers 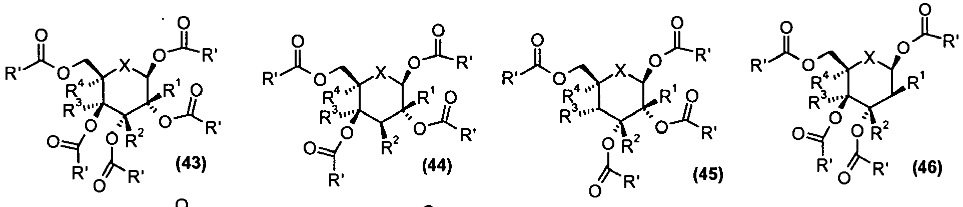 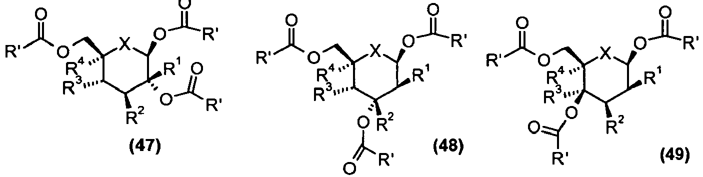 and other diastereoisomers
wherein R is defined as above; wherein R¹, R², R³, R⁴ and R⁵ are independently H alkyl, hydroxy, hydroxyalkyl -NR'R", SR"', halogeno; wherein R', R" and R"' are defined as above and wherein B is a nucleobase.

### Example 1

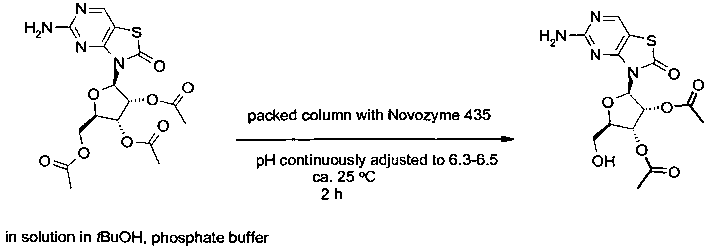

A 1 cm diameter filter Nutsche was charged with ca. 1 g Candida Antarctica Lipase Novozym 435. A solution of adduct (ca. 1 g dissolved into 9 mL t-butanol and 16 mL pH 7.0 phosphate buffer) was passed through the filter at ca. 1.6 mL/min (ca. 0.2 bar pressure) until completion. The pH of the filtered mixture was continuously maintained between 6.3 and 6.5 with a Na₂HPO₄ solution. The reaction was complete after ca. 2 h. The phases were then easily separated and the aqueous phase was extracted one time with ca. 20 mL 2-methyltetrahydrofuran. The combined organic phases were washed once with water and concentrated under reduced pressure to give the crude product in >90% yield and with < 1% over-hydrolysis by-product.

This continuous process of the present invention has several advantages over a batch process as described e.g. in WO05/121162, e.g., improved yield, faster reaction, continuous process possible for work-up, no more filtration, enzyme is easily recycled, increased throughput, reduced waste and, importantly, improved selectivity and minimized hydrolysis to the undesired monoacetate and tris-hydroxy compounds. The crude product was obtained in ca. 90% yield with 3-5% over-hydrolysis by the method as described in WO05/121162 while it can be obtained in yield higher than 90% with less than 1 % over-hydrolysis by-products in continuous mode.

## Claims

1. A process for the regioselective enzymatic hydrolysis of a substrate comprising passing a buffered solution through an immobilized enzyme in a continuous process, wherein the substrate is 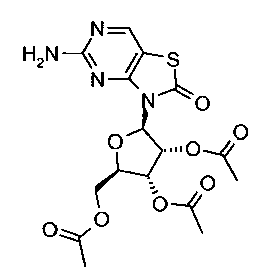 the enzyme is *Candida Antarctica Novozym* 435 Lipase;
the buffered solution has a pH of 5.5 to 7.5; and
the *Candida Antarctica* Lipase regioselectively hydrolyzes the ester at the 5' position of the substrate.

2. A process according to claim 1 wherein the substrate is in a buffered solution and the buffered solution is passed through the immobilized enzyme at a rate of 0.1 mL/min to 50 mL/min, or 0.5 mL/min to 10 mL/min

3. A process according to claim 1 or 2 wherein the buffered solution comprises a phosphate buffer, an ammonium buffer, a carbonate buffer, or an acetate buffer.

4. A process according to claim 1 or 2 wherein the buffered solution comprises a phosphate buffer.

## Patentansprüche

1. Verfahren zur regioselektiven enzymatischen Hydrolyse eines Substrats, umfassend eine gepufferte Lösung durch ein immobilisiertes Enzym in einem kontinuierlichen Verfahren durchzuleiten, bei dem das Substrat 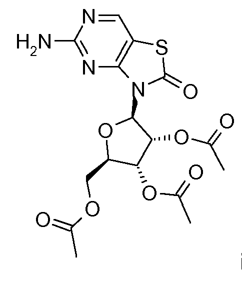 ist,
das Enzym *Candida antarctica* Lipase Novozym 435 ist;
die gepufferte Lösung einen pH von 5,5 bis 7,5 besitzt; und
die *Candida Antarctica* Lipase regioselektiv den Ester an der 5'-Position des Substrats hydrolysiert.

2. Verfahren nach Anspruch 1, wobei das Substrat in einer gepufferten Lösung vorliegt und die gepufferte Lösung durch das immobilisierte Enzym mit einer Rate von 0,1 ml/min bis 50 ml /min oder 0,5 ml /min bis 10 ml / min durchgeleitet wird.

3. Verfahren nach Anspruch 1 oder 2, wobei die gepufferte Lösung einen Phosphatpuffer, einen Ammoniumpuffer, einen Karbonatpuffer oder einen Acetatpuffer umfasst.

4. Verfahren nach Anspruch 1 oder 2, wobei die gepufferte Lösung einen Phosphatpuffer umfasst.

## Revendications

1. Processus pour l'hydrolyse enzymatique régiosélective d'un substrat, comprenant l'étape consistant à passer une solution tamponnée au travers d'un enzyme immobilisé dans un processus continu, dans lequel le substrat est 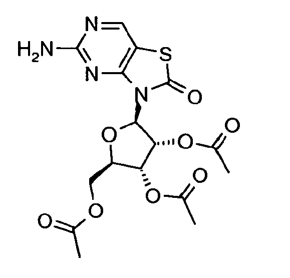 l'enzyme est la Lipase de *Candida Antarctica Novozyme* 435 ;
la solution tamponnée a un pH de 5,5 à 7,5 ; et
La Lipase de *Candida Antarctica* hydrolyse de façon régiosélective l'ester en position 5' du substrat.

2. Processus selon la revendication 1, dans lequel le substrat est dans une solution tamponnée et la solution tamponnée est passée au travers de l'enzyme immobilisé à une vitesse de 0,1 ml / mn à 50 ml / mn, ou de 0,5 ml / mn à 10 ml / mn.

3. Processus selon la revendication 1 ou la revendication 2, dans lequel la solution tamponnée comprend un tampon phosphate, un tampon ammonium, un tampon carbonate, ou un tampon acétate.

4. Processus selon la revendication 1 ou la revendication 2, dans lequel la solution tamponnée comprend un tampon phosphate.
